# EUROPÄISCHE PATENTSCHRIFT

(11) **EP 0 223 244 B1**
(45) Veröffentlichungstag und Bekanntmachung des Hinweises auf die Patenterteilung: **27.10.1993**
(21) Anmeldenummer: 86116025.7
(22) Anmeldetag: 18.11.1986
(51) Int. Cl.: G01N 33/49

(54) **Verfahren und Einrichtung zur Messung der Aggregation der Blutplättchen bzw. der Koagulation des Blutes**
Method and apparatus for measuring the platelet aggregation and the coagulation of blood
Procédé et dispositif de mesure de l'agrégation des plaquettes sanguines et de la coagulation du sang

(30) Priorität: 19.11.1985 DE 3541057
(43) Veröffentlichungstag der Anmeldung: 27.05.1987
(73) Patentinhaber: BAXTER DIAGNOSTICS INC., Deerfield, IL 60015 (US)
(72) Erfinder: Kratzer, Michael, Dr., D-8000 München 40 (DE)
(74) Vertreter: von Puttkamer, Nikolaus, Dipl.-Ing. Patentanwälte Haft, von Puttkamer Berngruber, Czybulka

(56) Entgegenhaltungen:
- GB-A- 2 096 329
- US-A- 3 486 859
- US-A- 3 720 097
- US-A- 3 911 728

## Beschreibung

Die Erfindung betrifft ein Verfahren zur Messung der Aggregation der Blutplättchen bzw. der Koagulation des Blutes nach dem Oberbegriff des Patentanspruches 1 und eine Einrichtung zur Durchführung dieses Verfahrens nach dem Oberbegriff des Patentanspruches 8, entsprechend GB-A-2 096 329.

Aus der DE-OS 24 06 484 ist ein Verfahren zum Messen der Koagulation des Blutes bekannt, bei dem Blut aus einem Vorratsgefäß in ein mit einem Zylinder verbundenes Rohr dadurch einsaugbar ist, daß ein in dem Zylinder vorgesehener Kolben zunächst so bewegt wird, daß er eine Saugwirkung im Rohr entfaltet. Der Kolben ist mit einem Antrieb verbunden und durch diesen anschließend derart hin- und herbewegbar, daß das in das Rohr eingesaugte Blutvolumen ebenfalls Hin- und Herbewegungen im Rohr ausführt. Bei diesen Bewegungen wird das zwischen dem Kolben und dem Blutvolumen befindliche Gas aufeinanderfolgend expandiert und komprimiert. Die Druckveränderung dieses Gases wird als ein Maß für die Viskositätsänderung bzw. Koagulation des Blutes gemessen.

Ein Problem eines derartigen Verfahrens besteht darin, daß die Koagulation des Blutes nicht unter physiologischen Bedingungen gemessen werden kann, weil die Ausbildung eines Thrombus im Rohr durch die auf ihn einwirkenden Drücke beeinflußt wird. Zudem ist bei diesem Verfahren die sich als Maß für die Koagulation im Gas ergebende Druckveränderung nur relativ ungenau und aufwendig meßbar. Das in diesem Zusammenhang verwendete Druckmeßgerät, das aus einem einseitig mit dem Gasraum zwischen dem Kolben und dem eingesaugten Blutvolumen in Verbindung stehende U-förmigen Glasrohr besteht, ist umständlich handhabbar, weil stets darauf geachtet werden muß, daß die in dem U-förmigen Glasrohr befindliche Flüssigkeit, bei der es sich z.B. um Quecksilber handelt, nicht aus dem Glasrohr heraustritt. Dies könnte beispielsweise dann eintreten, wenn die gesamte Meßeinrichtung z.B. bei einer unsachgemäßen Handhabung auf den Kopf gestellt würde. Da derartige Handhabungen insbesondere beim Versand häufig vorkommen, kann das Quecksilber nicht direkt vom Hersteller, sondern erst dann eingefüllt werden, wenn die gesamte Meßeinrichtung an ihrem Bestimmungsort, beispielsweise in einem Labor, aufgestellt worden ist. Der Ablesevorgang an der am U-förmigen Glasrohr ausgebrachten Skala ist relativ zeitaufwendig und ungenau.

Aus der GB-A 2 096 329 geht ein Verfahren hervor, bei dem durch eine Öffnung Blut in eine Kapillare eingesaugt wird. Als Druckreservoir dient dabei ein Gefäß, in dem durch eine Vakuumpumpe und durch steuerbare Ventile der erforderliche Unterdruck erzeugt wird. Als Druckmeßgerät wird, wie bei der DE-OS 24 06 484 eine Flüssigkeitssäule in einem U-förmigen Bereich der Kapillare verwendet.

Aus der US-A-3 486 859 gehen ein Verfahren und eine Einrichtung der eingangs genannten Art hervor. Dabei wird ein Zylinder in einem Kolben bewegt, wobei in den dem Kolben vorgelagerten Raum Blut durch eine Kapillare eingesaugt wird. Der Druck im Raum zwischen dem Kolben und dem eingesaugten Blut wird durch eine Druckzelle gemessen und durch eine Spannungsmeßeinrichtung angezeigt. Messungen unter physiologischen Bedingungen sind nicht möglich, weil sich bei einer Bewegung des Kolbens und bei der Ausbildung eines Thrombus in der Kapillare der Druck im Raum ändert, so daß die Ausbildung des Thrombus durch diese Druckänderung beeinflußt wird.

Die Aufgabe der vorliegenden Erfindung besteht daher darin, ein relativ genaues Verfahren zur Messung der Aggregation der Blutplättchen bzw. der Koagulation des Blutes, das Messungen unter physiologischen Bedingungen ermöglicht, und eine Einrichtung zur Durchführung dieses Verfahrens anzugeben, die relativ einfach handhabbar ist.

Diese Aufgabe wird durch ein Verfahren, das durch die in dem kennzeichnenden Teil des Patentanspruches 1 angegebenen Merkmale gekennzeichnet ist, und durch eine Einrichtung gelöst, die durch die in dem kennzeichnenden Teil des Patentanspruches 8 enthaltenen Merkmale gekennzeichnet ist.

Ein wesentlicher Vorteil der Erfindung besteht darin, daß die Messung der Aggregation der Blutplättchen und der Koagulation des Blutes erstmals unter physiologischen Bedingungen, d.h. also unter Bedingungen meßbar ist, bei denen auf das Blut ein vorbestimmter konstanter Druck ausgeübt wird. Dadurch ist es möglich, die im Körper auftretenden Vorgänge, die beispielsweise beim Bluten einer Schnittwunde o.dgl. ablaufen, genau nachzubilden.

Ein weiterer Vorteil der Erfindung besteht darin, daß genauere, reproduzierbare Mesungen vorgenommen werden können.

Vorteilhafterweise können mit der Erfindung auch Fälle simuliert werden, bei denen sich der auf das Blut einwirkende Druck während einer vorgegebenen Zeitperiode in einer genau vorbestimmten Weise verändert, z.B. nach einer vorgegebenen zeitabhängigen Funktion zuerst zunimmt und danach wieder abnimmt. Derartige Druckverläufe können auch im menschlichen Körper (Blutdruckschwankungen) auftreten. Somit ist durch eine gezielte zeitabhängige Druckveränderung die Nachbildung bestimmter Krankheitssymptome etc. erstmals möglich.

Ein weiterer Vorteil der Erfindung besteht darin, daß sie den Aufbau einer äußerst funktionsgerechten, funktionssicheren, kompakten und einfachen Einrichtung ermöglicht.

Das erfindungsgemäße Verfahren und die erfindungsgemäße Einrichtung eignen sich vorzugsweise zur Ausführung von On-Line-Messungen an Patienten, die sich beispielsweise nach einer Nierentransplantation o.dgl. in einer kritischen Phase befinden. Computergesteuert können dann in einem zeitlichen Abstand von z.B. etwa 10 Minuten Messungen automatisch vorgenommen werden, so daß die Änderungsrate der Aggregation der Blutplättchen bzw. der Koagulation bestimmt werden kann, die z.B. ein Maß für krankhafte Verläufe, wie z.B. Verbrauchskoagulopatie darstellen.

Im folgenden werden die Erfindung und deren Ausgestaltungen im Zusammenhang mit den Figuren näher erläutert. Es zeigt:
- Fig. 1: den Aufbau einer erfindungsgemäßen Einrichtung zur Messung der Aggregation bzw. Koagulation des Blutes;
- Fig. 2: typische Druckverläufe;
- Fig. 3: eine Weiterbildung der Erfindung; und
- Fig. 4: eine Weiterbildung der Erfindung zur Durchführung von On-Line-Messungen.

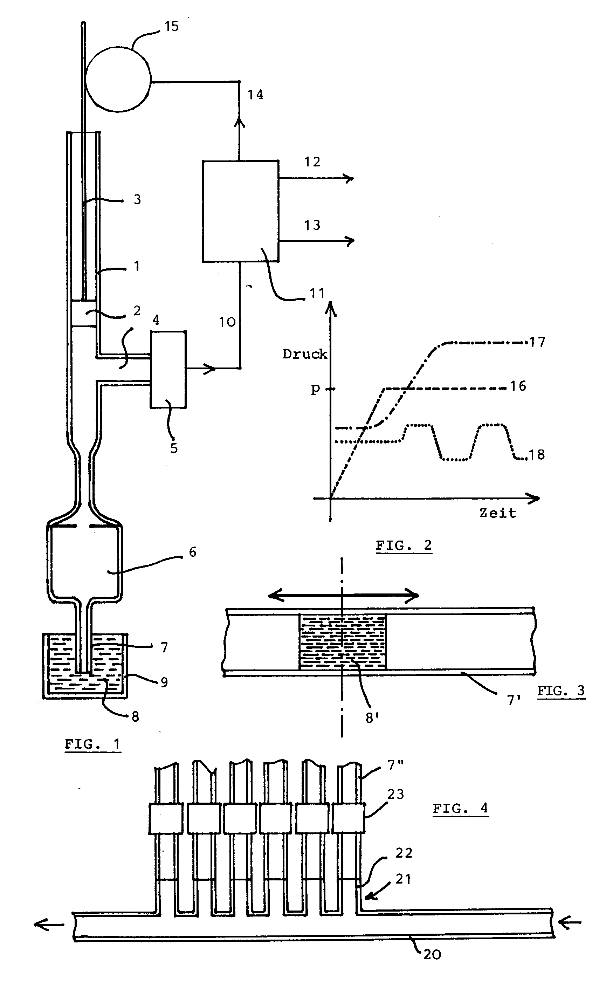

In der Fig. 1 befindet sich das Blut 8, dessen Blutplättchen-Aggregation bzw. Koagulation gemessen werden soll, in einem Becher bzw. Gefäß 9. In dieses Gefäß 9 ragt eine Kapillare 7 hinein, die zweckmäßigerweise mit einem Reservoir 6 zur Blutaufnahme in Verbindung steht. Dieses Reservoir 6 ist mit einem Zylinder 1, bei dem es sich vorzugsweise um ein Glasrohr o.dgl. handelt, verbunden. In dem Zylinder 1 ist ein Kolben 2 derart angeordnet, daß er in axialer Richtung des Zylinders 1 bewegbar ist. Vorzugsweise ist der Kolben 2 mit einer Kolbenstange 3 verbunden, an der außerhalb des Zylinders 1 zur axialen Bewegung des Kolbens 2 ein Antriebselement 15 angreift. Bei diesem handelt es sich vorzugsweise um einen Schrittmotor, der z.B. über ein nicht dargestelltes Ritzel in einen Bereich (nicht dargestellt) der Kolbenstange 3 eingreift, der als Zahnstange ausgebildet ist. Der Raum zwischen dem Kolben 2 und der Kapillare 7 steht über eine Zweigleitung 4 mit einem Drucksensor 5 in Verbindung, durch den der in dem Raum herrschende Druck meßbar ist. Der Drucksensor 5 erzeugt ein dem gemessenen Druck proportionales Ausgangssignal, das über eine Leitung 10 einem Rechner oder Microprozessor 11 o.dgl. zugeführt wird. Der Microprozessor 11 kann in Abhängigkeit von einem ihm eingegebenen Druck-Sollwert an der Ausgangsleitung 14 ein Steuersignal erzeugen, das dem Antrieb 15 zugeführt wird. Der Drucksensor 5, die Leitung 10, der Microprozessor 11, die Ausgangsleitung 14 und der Antrieb 15 stellen einen negativen Rückkopplungszweig dar, durch den der Druck in dem Raum zwischen dem Kolben 2 und dem in die Kapillare 7 eingeströmten Blut auf einem vorgegebenen Wert gehalten wird. Dieser Vorgang wird im folgenden im einzelnen näher erläutert. Dabei wird zunächst davon ausgegangen, daß der Druck auf einem konstanten Soll -Druckwert p gehalten wird.

Wenn die Kapillare 7 in das im Gefäß 9 befindliche Blut eintaucht und der Kolben 2 durch den Antrieb 15 in Fig. 1 nach oben bewegt wird, ändert sich der durch den Drucksensor 5 gemessene Druck im Raum zwischen dem in der Kapillare 7 einströmenden Blut und dem Kolben 2 so lange an, bis der dem Microprozessor 11 eingegebene Soll-Druckwert p erreicht ist. Dieser Vorgang entspricht in Fig. 2 dem ansteigenden Bereich der Kurve 16. nach Erreichen des Soll-Druckwertes p beginnt der Microprozessor 11 den Druck in dem genannten Raum derart zu regeln, daß er konstant auf dem Soll-Druckwert gehalten wird. Dies bedeutet, daß das Blut durch die Kapillare 7 unter genau reproduzierbaren und physiologischen Bedingungen fließt, da auch im menschlichen oder tierischen Körper beim Blutaustritt der Blutdruck im wesentlichen konstantbleibt. Beim Einfließen von Blut in die Kapillare 7 steigt der am Sensor 5 angezeigte Druck jeweils geringfügig um einen Wert Δp. Diese Differenz Δp wird aber dadurch auf Null gebracht, daß der Microprozessor 11 den Schrittmotor bzw. Antrieb 15 in Abhängigkeit von der Differenz Δp ansteuert. Dabei ist die Bewegung des Kolbens 2 eine Funktion der durch die Kapillare 7 einströmenden Blutmenge. Da der Durchmesser des Zylinders 1 und der zurückgelegte Weg des Kolbens dem Microprozessor 11 bekannt sind, kann er am Ausgang 12 die Blutflußmenge direkt anzeigen. Die Anderung der Blutflußmenge, die in Volumen/Zeit angegeben wird, stellt ein direktes Maß für die Aggregation der Blutplättchen bzw. Koagulation des Blutes dar. Mit dem Ausgang 12 ist ein Anzeige- bzw. Aufzeichnungsgerät (nicht dargestellt) verbunden, das den Verlauf der Änderung der Blutflußmenge für spätere oder direkte Auswertungen aufzeichnet. Am Ausgang 13 kann der Druck p für Auswertvorgänge angezeigt werden. Beispielsweise werden die Signale der Ausgänge 12 und 13 durch einen Koordinatenschreiber in der Form von zeitabhängigen Kurven aufgezeichnet.

Es ist von ganz wesentlicher Bedeutung, daß der Druck im Raum zwischen dem Kolben 2 und dem einströmenden Blut genau auf einen vorgegebenen Druckwert z.B. konstant gehalten wird. Wenn der Druck konstant gehalten wird, kann das Blut, wie dies schon angesprochen wurde, unter physiologischen Bedingungen, d.h. bei konstantem Druck, fließen. Dies bedeutet, daß ein Thrombus sich in der Kapillare 7 ungehindert ausbilden kann, wie dies z.B. auch bei Blutungsvorgängen an einer Schnittwunde der Fall wäre. Beim genannten Stand der Technik würde dagegen bewirkt, daß der Druck zwischen dem Kolben und dem einströmenden Blut bei der Ausbildung eines Thrombus erheblich ansteigen würde, so daß der sich ausbildende Thrombus aus dem Bereich der Kapillare 2 regelrecht "herausgerissen" würde. Aus diesem Grunde können dann die nach dem "Abreißen" ablaufenden Vorgänge durch die Einrichtungen des Standes der Technik nicht mehr gemessen werden.

Es ist das Verdienst der vorliegenden Erfindung, daß erstmals darauf geachtet wurde, daß der Druck, mit dem das Blut in die Kapillare eingesaugt wird, auf einem vorgegebenen Wert gehalten wird, weil dies für eine ungestörte oder naturgemäße Ausbildung eines Thrombus in der Kapillare von wesentlicher Bedeutung ist.

Zur naturgetreuen Nachbildung von z.B. im menschlichen oder tierischen Körper ablaufenden Vorgängen (z.B. Streß), bei denen sich der Blutdruck zeitabhängig ändert, kann gemäß den Kurven 17 und 18 der Fig. 2 der Druck im Raum zwischen dem Kolben 2 und dem einströmenden Blut auch nach einer genau vorgegebenen Funktion geändert werden. Beispielsweise wird gemäß der Kurve 17 der Druck in Abhängigkeit von der Zeit von einem ersten Druckwert auf einen zweiten Druckwert erhöht. Der Kurve 18 entsprechend kann der Druck auch zeitvariabel zwischen einem ersten Wert und einem zweiten Wert schwanken.

Aus Fig. 3 geht eine Weiterbildung der Erfindung hervor, bei der das in die Kapillare 7' eingeströmte Blut nicht in einer Richtung, sondern durch eine entsprechende Bewegung des Kolbens 2 hin- und herbewegt wird. Beispielsweise wird dies dadurch erreicht, daß eine eingesaugte kleinere Blutmenge durch den vom Antrieb 15 in Hin- und Herbewegungen versetzte Kolben2 in der Kapillare 7' hin- und herbewegt wird. Von entscheidender Bedeutung ist es jedoch auch in diesem Fall, daß bei jeder Bewegungsrichtung der Druck durch die negative Rückkopplung auf einen Soll-Druckwert geregelt wird, der entweder konstant ist (Kurve 16, Fig. 2) oder einer ganz genau vorgegebenen Druck/Zeit-Funktion (Kurven 17, 18, Fig. 2) folgt. Dabei ist es von Vorteil, lediglich eine kleine Blutmenge 8' in die Kapillare 7' einzusaugen, die dann die Hin- und Herbewegungen ausführt. Infolge der gezielten Verminderung von schädlichen Drücken wird auch in diesem Fall verhindert, daß ein sich ausbildender Thrombus durch zu hohe Drücke von der Innenwand der kapillare 7' losgerissen wird.

Im Falle der Ausführungsform der Fig. 3, bei der nur eine kleine Blutmenge eingesaugt wird, kann das Reservoir 6 (Fig. 1) entfallen.

Die Hin- und Herbewegung des Kolbens 2 wird vorzugsweise durch Bewegung der Kolbenstange 3 durch Umkehrung der Drehrichtung des Antriebs 15 erreicht, nachdem der Kolben zuvor einen vorgegebenen Hub zur Aufnahme der kleinen Blutmenge 8 ausgeführt hat.

Im Zusammenhang mit der Art des verwendeten Antriebs 15 wird darauf hingewiesen, daß die Bewegung des Kolbens in einer Richtung oder in beiden Richtungen in vorteilhafter Weise durch einen Schrittmotor erzielt wird. Dieser Schrittmotor kann genau durch ein vom Microprozessor 11 abgegebenes digitales Steuersignal zur Druckregelung gesteuert werden. Dabei können die einzelnen Schritte des Motors, die einer minimalen Bewegung des Kolbens entsprechen, durch Auswahl des Motors beliebig fein gewählt werden. Es ist jedoch auch denkbar, einen Motor analog anzusteuern. Die Kopplung zwischen der Kolbenstange 3 und dem Motor 15 kann auch über einen Reibmechanismus oder ein anderes geeignetes Getriebe ausgeführt werden.

Aus der Fig. 4 geht eine Weiterbildung bzw. Anwendung der Erfindung hervor, mit deren Hilfe an kritischen Patienten in einer einfachen Weise On-Line-Untersuchungen vorgenommen werden können, wie sie beispielsweise nach Transplantationen wünschenswert sind. In diesem Fall kann nämlich die Erfassung der Änderung der Aggregation der Blutplättchen bzw. die Änderung der Koagulation des Blutes in Abhängigkeit von der Zeit von großer Wichtigkeit sein. Ein von einem Patienten kommendes Katheterrohr 20 o.dgl., durch das dem Patienten laufend Blut entnommen und wieder zugeführt wird, weist hintereinander mehrere Abzweigungen 21 (z.B. T-Stücke) auf, die jweils über Abzweigleitungen 22 und ein beispielsweise elektromechanisch betätigtes Absperrventil 23 zu einer Kapillare 7'' führt, die mit einer vorliegenden Meßeinrichtung verbunden ist. Bei den Abzweigungsleitungen 22 kann es sich beispielsweise jeweils um einen Schlauch und bei den Absperrventilen 23 um eine elektromagnetisch betätigte Schlauchklemme handeln. Die Abpserrventile werden in zeitlicher Aufeinanderfolge, z.B. im zeitlichen Abstand von 15 Minuten, derart geöffnet, daß immer nur ein einziges Ventil, das gerade Blut zur Messung freigeben soll, geöffnet ist. Nach dem Öffnen eines Ventiles 23 fließt aufgrund der durch den Microprozessor 11 bewirkten Kolbenbewegung Blut in die Kapillare 7''. Der Meßvorgang entspricht dem oben erläuterten Verfahren, gemäß dem Blut in einer Richtung in das Reservoir 6 (Fig. 1) eingesaugt wird.

Bei der Meßung gemäß Fig. 3 erfolgt nach dem Einsaugen des kleinen Blutvolumens in eine Kapillare 7'' zweckmäßigerweise durch das entsprechende Ventil 23 jeweils eine Belüftung, so daß die Hin- und Herbewegung in der Kapillare 7' möglich ist. Bei einem Meßabstand von 15 Minuten kann bei der dargestellten Ausführungsform mit den sechs Abzweigleitungen 22 die Änderung der Aggregation der Blutplättchen bzw. der Koagulation des Blutes über einen Zeitraum von 1,5 Stunden erfaßt werden. Dadurch, daß die jeweils benutzten Kapillaren 7'' automatisch durch neue Kapillaren ersetzt werden, kann die Untersuchung bei richtiger Steuerung der Ventile 23 endlos durchgeführt werden. Die Steuerung der Ventile 23 erfolgt vorteilhafterweise ebenfalls durch denMicroprozessor 11. Es ist auch denkbar, die beschriebene On-Line-Messung mit einer einzigen Kapillare durchzuführen, die nach jeder erfolgten Messung vorzugsweise automatisch ausgewechselt wird.

Die Kapillare 7, 7', 7'' kann in der Form eines einfachen Wegwerfteiles beispielsweise aus einem Kunststoffmaterial ausgebildet und am Reservoir 6 (Fig. 1) bzw. direkt am Zylinder 1 (Fig. 3) befestigt sein.

Der Zylinder 1 und der Kolben 2 können besonders vorteilhaft und billig durch eine auf dem Markt befindliche Spritze realisiert werden.

Die Empfindlichkeit der Regelschleife 5, 10, 11, 14, 15, 3, 2 wird durch die Größe des Volumens des Raumes zwischen dem Kolben 2 und dem eingesaugten Blut in der Kapillare 7, 7', 7'' bzw. dem Reservoir 6 bestimmt. Dies bedeutet, daß durch die Dimensionierung dieses Raumes Einfluß auf die Genauigkeit der Druckregelung genommen werden kann.

In das Gefäß 9 können vor Beginn des Meßvorganges Substanzen von außen eingeführt werden, die die Gerinnung des Blutes unter definierten Bedingungen einleiten. Z.B. kann hierfür Thromoplastin zur Messung des exogenen Wegen der Gerinnung zugeführt werden.

Zur Messung der Aggregation der Blutplättchen wird vor Beginn des Meßvorganges dem Blut ein die Plättchen des Blutes aggregierendes Mittel zugegeben. Derartige Mittel sind in der DE-PS 32 47 815 offenbart.

Zusammenfassend wird ausgeführt, daß es im Zusammenhang mit der vorliegenden Erfindung von entscheidender Bedeutung ist, daß im Gegensatz zu allen früheren, bekannten Messungen der Druck als eine unabhängige, physikalische Größe vorgegeben wird und der Volumenstrom (Volumen pro Zeit) als abhängige physikalische Größe gemessen wird. Dieses Ziel kann nur mit Hilfe der erstmals vorgesehenen Rückkopplungsschleife erreicht werden, die dafür sorgt, daß vorgegebene Druck-Sollwerte in einer einfachen Weise äußerst genau eingehalten werden. Dabei können die vorgegebenen Soll-Druckwerte konstant sein oder sich in einer speziell vorgegebenen Weise zeitabhängig ändern. Durch die Vermeidung schädlicher Drücke durch vorgegebene Grenz-Druckwerte wird zuverlässig sichergestellt, daß die Ausbildung eines Thrombus naturgemäß erfolgen kann und nicht durch Druckeinwirkungen schädlich beeinflußt wird, wie dies bei den bekannten Meßverfahren der Fall ist. Ein weiterer Vorteil besteht darin, daß durch eine gezielte und kontrollierte Drucksteigerung erstmals der kritische Druck bestimmt werden kann, der zur Ablösung eines Thrombus führt.

## Patentansprüche

1. Verfahren zur Messung der Aggregation der Blutplättchen bzw. der Koagulation des Blutes, wobei das Blut unter Beibehaltung eines Soll-Druckwertes in eine Kapillare eingesaugt wird, und als Maß für die Aggregation bzw. Koagulation die Blutflußmenge erfaßt wird, **dadurch gekennzeichnet**, daß das Blut mit Hilfe eines in einem mit, der Kapillare (7) verbundenen Zylinder (1) bewegbaren Kolbens (2) eingesaugt wird, daß der Druck im Raum zwischen dem Kolben und dem eingesaugten Blut gemessen wird, daß über eine Rückkopplung mit der Druckmessung, der Kolben (2) durch einen Antrieb (15) bewegt wird, derart, daß der Soll-Druckwert im Raum gehalten wird, und daß die Bewegung des Kolbens (2) als Maß für die Blutflußmenge herangezogen wird.

2. Verfahren nach Anspruch 1, dadurch gekennzeichnet, daß der Kolben (2) nur in einer Richtung bewegt wird.

3. Verfahren nach Anspruch 1, dadurch gekennzeichnet, daß der Kolben (2) nach dem Einsaugen einer Blutmenge in die Kapillare (7') hin- und herbewegt wird.

4. Verfahren nach einem der Ansprüche 1 - 3, dadurch gekennzeichnet, daß der Soll-Druckwert konstant ist.

5. Verfahren nach einem der Ansprüche 1 - 3, dadurch gekennzeichnet, daß sich der Soll-Druckwert in Abhängigkeit von der Zeit nach einer vorgegebenen Funktion (17, 18) ändert und daß die zeitabhängigen Druckwerte in Tabellen eines Rechners (12) gespeichert sind.

6. Verfahren nach einem der Ansprüche 1 bis 5, dadurch gekennzeichnet, daß einem Patienten kontinuierlich Blut über ein Katheterrohr (20) entnommen wird und daß in vorgegebenen Zeitintervallen dem Katheterrohr (20) Blut zur Bestimmung der Aggregations- bzw. Koagulationsänderung entnommen und gemessen wird.

7. Verfahren nach einem der Ansprüche 1 bis 6, dadurch gekennzeichnet, daß dem in die Kapillare (7, 7') einzuführenden Blut von außen bestimmte Substanzen zugeführt werden, die im Blut ablaufende Vorgänge, z.B. Aggregation, Gerinnung, beeinflussen.

8. Einrichtung zur Durchführung des Verfahrens nach einem der Ansprüche 1 bis 7, bei der Blut in eine Kapillare durch eine Einrichtung zur Druckerzeugung einsaugbar ist und eine Einrichtung zur Erfassung der Blutflußmenge vorgesehen ist, **dadurch gekennzeichnet**, daß die Einrichtung zur Druckerzeugung einen durch einen Antrieb (15) bewegbaren Kolben (2) in einem mit der Kapillare (7) verbundenen Zylinder (1) aufweist, daß die Einrichtung zur Erfassung der Blutflußmenge einen Drucksensor (5), der den im Raum zwischen dem Kolben und dem eingesaugten Blut herrschenden Druck ermittelt, und eine Rückkopplung zwischen dem Drucksensor (5) und dem Antrieb (15) umfaßt, durch die der Kolben (2) so bewegbar ist, daß der Druck im Raum auf einem Soll-Druckwert gehalten ist und daß ein Rechner (11) vorgesehen ist, der die Bewegung des Kolbens (2) in eine der Blutflußmenge entsprechende Größe umwandelt.

9. Einrichtung nach Anspruch 8, dadurch gekennzeichnet, daß als Antrieb (15) ein Schrittmotor vorgesehen ist, der eine mit dem Kolben (2) verbundene Kolbenstange (3) betätigt.

10. Einrichtung nach Anspruch 8 oder 9, dadurch gekennzeichnet, daß der Zylinder (2) und der Kolben (3) Teile einer Spritze sind.

11. Einrichtung nach einem der Ansprüche 8 bis 9, dadurch gekennzeichnet, daß zwischen der Kapillare (7) und dem Kolben (2) ein Blutreservoir (6) angeordnet ist.

12. Einrichtung nach einem der Ansprüche 8 bis 11, dadurch gekennzeichnet, daß zur Blutentnahme wenigstens eine Kapillare (7'') über eine Ventileinrichtung (23) mit einem Katheterrohr (20) verbunden ist.

13. Einrichtung nach Anspruch 12, dadurch gekennzeichnet, daß zur zeitlich beabstandeten Blutentnahme mehrere Kapillaren(7'') jeweils über eine Ventileinrichtung (23) mit dem Katheterrohr (20) verbunden sind.

14. Einrichtung nach einem der Ansprüche 8 bis 13, dadurch gekennzeichnet, daß die Kapillare (7, 7', 7'')die Form eines mit dem Zylinder (1) oder dem Reservoir (6) verbundenen Einwegteiles aufweist.

15. Einrichtung nach Anspruch 14, dadurch gekennzeichnet, daß das Einwegteil automatisch auswechselbar ist.

16. Einrichtung nach einem der Ansprüche 8 bis 15, dadurch gekennzeichnet, daß der Rechner (11) eine Druckwerte beinhaltende Tabelle zur Berechnung des den Antrieb (15) steuernden Signals aufweist.

## Claims

1. A process for the measurement of the aggregation of blood platelets and respectively the coagulation of blood, whereby the blood is sucked, whilst maintaining a set pressure value, into a capillary, and the amount of blood flow is detected as a measure for the aggregation and coagulation respectively,
**characterised in that** the blood is sucked in with the aid of a plunger (2), which can move in a cylinder (1) connected to the capillary (7),
**in that** the pressure in the space between the plunger and the sucked-in blood is measured,
**in that** the plunger (2) is moved by a drive (15) via a feedback with the pressure measurement so that the set pressure value in the space is maintained,
**and in that** the movement of the plunger (2) is used as a measure for the amount of blood flow.

2. A process according to Claim 1,
**characterised in that** the plunger (2) is moved only in one direction.

3. A process according to Claim 1,
**characterised in that** after the suction of a quantity of blood into the capillary (7') the plunger (2) is moved backwards and forwards.

4. A process according to one of Claims 1 to 3,
**characterised in that** the set pressure value is constant.

5. A process according to one of Claims 1 to 3,
**characterised in that** the set pressure value changes as a function of time according to a predetermined function (17, 18),
**and in that** the time-dependent pressure values are stored in tables of a computer (12).

6. A process according to one of Claims 1 to 5,
**characterised in that** blood is continually taken from a patient via a catheter tube (20),
**and in that** blood is taken at predetermined intervals of time from the catheter tube (20) and measured to determine the alteration in aggregation and coagulation respectively.

7. A process according to one of Claims 1 to 6,
**characterised in that** determined substances, which influence the processes occurring in the blood, e.g. aggregation, coagulation, are supplied from outside to the blood to be introduced into the capillary (7, 7').

8. Apparatus to perform the process according to one of Claims 1 to 7, in which blood can be sucked into a capillary by a device for the generation of pressure,
**characterised in that** the device for the generation of pressure comprises a plunger (2), which can be moved by a drive (15), in a cylinder (1) connected to the capillary (7),
**in that** the device for detecting the amount of blood flow comprises a pressure sensor (5), which determines the pressure prevailing in the space between the plunger and the sucked-in blood, and a feedback between the pressure sensor (5) and the drive (15), by which the plunger (2) can be moved so that the pressure in the space is kept at a set pressure value,
**and in that** a computer (11) is provided, which converts the movement of the plunger (2) into a magnitude corresponding to the amount of blood flow.

9. Apparatus according to Claim 8,
**characterised in that** a stepping motor, which is operated with a rod (3) connected to the plunger (2), is provided as a drive (15).

10. Apparatus according to Claim 8 or 9,
**characterised in that** the cylinder (2) and the plunger (3) are parts of a syringe.

11. Apparatus according to one of Claims 8 to 9,
**characterised in that** between the capillary (7) and the plunger (2) is disposed a blood reservoir (6).

12. Apparatus according to one of Claims 8 to 11,
**characterised in that** for the removal of blood at least one capillary (7'') is connected via a valve mechanism (23) to a catheter tube (20).

13. Apparatus according to Claim 12,
**characterised in that** several capillaries (7'') are connected via a valve mechanism (23) to the catheter tube (20) for the removal of blood spaced in time.

14. Apparatus according to one of Claims 8 to 13,
**characterised in that** the capillary (7, 7', 7'') has the form of a one-way part connected to the cylinder (1) or the reservoir (6).

15. Apparatus according to Claim 14,
**characterised in that** the one-way part is automatically replaceable.

16. Apparatus according to one of Claims 8 to 15,
**characterised in that** the computer (11) comprises a table containing pressure values to calculate the signal controlling the drive (15).

## Revendications

1. Procédé de mesure de l'agrégation plaquettaire ou de la coagulation du sang, selon lequel on aspire le sang dans un tube capillaire en maintenant une pression de consigne et on mesure le débit de sang en tant que grandeur indiquant l'agrégation ou la coagulation, procédé caractérisé par le fait qu'on aspire le sang au moyen d'un piston (2) mobile dans un cylindre (1) connecté au tube capillaire (7), par le fait qu'on mesure la pression à l'intérieur de la chambre entre le piston et le sang aspiré, par le fait que, par une rétroaction avec la mesure de pression, on déplace le piston (2) au moyen d'un dispositif d'entraînement (15) de manière à maintenir la pression de consigne dans la chambre et par le fait qu'on utilise le déplacement du piston (2) comme grandeur indiquant le débit de sang.

2. Procédé selon la revendication 1, caractérisé par le fait qu'on déplace le piston (2) dans une seule direction.

3. Procédé selon la revendication 1, caractérisé par le fait qu'après avoir aspiré une certaine quantité de sang dans le tube capillaire (7') on déplace le piston (2) suivant un mouvement de va-et-vient.

4. Procédé selon la revendication 1 - 3, caractérisé par le fait que la pression de consigne est constante

5. Procédé selon l'une des revendications 1 - 3, caractérisé par le fait que la pression de consigne varie dans le temps suivant une fonction (17, 18) prédéterminée et par le fait que les pressions en fonction du temps sont mémorisées dans des tables d'un calculateur (12).

6. Procédé selon l'une des revendications 1 à 5, caractérisé par le fait qu'on prélève en continu du sang à un patient par l'intermédiaire d'un catheter (20) et par le fait que l'on prélève à intervailes prédéterminés du sang sur le tube de catheter (20) aux fins de déterminer la variation de l'agrégation ou de la coagulation.

7. Procédé selon l'une des revendications 1 à 6, caractérisé par le fait qu'on ajoute de l'extérieur au sang à introduire dans le tube capillaire (7, 7') certaines substances qui influent sur certains phénomènes qui se déroulent dans le sang, comme par exemple l'agrégation, la coagulation.

8. Dispositif pour la mise en oeuvre du procédé selon l'une des revendications 1 à 7, dans lequel du sang peut être aspiré dans un tube capillaire au moyen d'un dispositif de génération de pression et dans lequel il est prévu un dispositif de mesure du débit de sang, caractérisé par le fait que le dispositif de génération de pression présente un piston (2) qui, par un mécanisme d'entraînement (15), peut être déplacé dans un cylindre (1) connecté au tube capillaire (7), par le fait que le dispositif de mesure du débit de sang comprend un capteur de pression (5) qui mesure la pression à l'intérieur de la chambre entre le piston et le sang aspiré ainsi qu'un dispositif de rétroaction entre le capteur de pression (5) et le dispositif d'entraînement (15) au moyen duquel le piston (2) est déplacé de manière à maintenir la pression dans la chambre à une valeur de consigne, par le fait qu'il est prévu un calculateur (11) qui transforme le déplacement du piston (2) en une grandeur correspondant au débit de sang.

9. Dispositif selon la revendication 8, caractérisé par le fait qu'il est prévu comme dispositif d'entraînement (15) un moteur pas à pas qui actionne une tige de piston (3) liée au piston (2).

10. Dispositif selon la revendication 8 ou 9, caractérisé par le fait que le cylindre (1) et le piston (2) font partie d'une seringue.

11. Dispositif selon l'une des revendications 8 à 9, caractérisé par le fait qu'un réservoir à sang (6) est disposé entre le tube capillaire (7) et le piston (2).

12. Dispositif selon l'une des revendications 8 à 11, caractérisé par le fait que, pour le prélèvement de sang, au moins un tube capillaire (7'') est relié à un tube de catheter (20) par l'intermédiaire d'un système de valve (23).

13. Dispositif selon la revendication 12, caractérisé par le fait que, pour le prélèvement de sang espacé dans le temps, plusieurs tubes capillaires (7'') sont reliés au tube de catheter (20) chacun par l'intermédiaire d'un système de valve (23).

14. Dispositif selon l'une des revendications 8 à 13, caractérisé par le fait que le tube capillaire (7, 7', 7'') se présente sous la forme d'un élément jetable connecté au cylindre (1) ou au réservoir (6).

15. Dispositif selon la revendication 14, caractérisé par le fait que l'élément jetable peut être remplacé de manière automatique.

16. Dispositif selon l'une des revendications 8 à 15, caractérisé par le fait que le calculateur (11) comporte une table avec des pressions pour le calcul du signal de commande du dispositif d'entraînement (15).
